# EUROPEAN PATENT SPECIFICATION

(11) **EP 4 489 842 B1**
(45) Date of publication and mention of the grant of the patent: **18.11.2026**
(21) Application number: 23714420.9
(22) Date of filing: 10.03.2023
(51) Int. Cl.: A61N 1/05, A61N 1/375, A61N 1/39

(54) **IMPLANTABLE MEDICAL LEAD WITH SHIELD**
IMPLANTIERBARE MEDIZINISCHE LEITUNG MIT ABSCHIRMUNG
FIL MÉDICAL IMPLANTABLE AVEC BLINDAGE

(30) Priority: 11.03.2022 US 202263269180 P
(43) Date of publication of application: 15.01.2025
(73) Proprietor: Medtronic, Inc., Minneapolis, Minnesota 55432 (US)
(72) Inventor: NIKOLSKI, Vladimir P., Mounds View, Minnesota 55112 (US); WILLIAMS, Dina L., Mounds View, Minnesota 55112 (US); MARSHALL, Mark T., Mounds View, Minnesota 55112 (US); CLEMENS, William J., Mounds View, Minnesota 55112 (US); HOFFMAN, Matthew J., Mounds View, Minnesota 55112 (US)
(74) Representative: Zimmermann & Partner Patentanwälte mbB
(86) International application number: PCT/US2023/014968
(87) International publication number: WO 2023/172732

(56) References cited:
- US-A1- 2018 133 458
- US-A1- 2018 326 202
- US-A1- 2019 117 972
- US-A1- 2021 268 297

## Description

This application claims the benefit of U.S. Provisional Patent Application Serial No. 63/269,180, filed 11 March 2022.

### TECHNICAL FIELD

The present application relates to implantable medical leads and, more particularly, implantable medical leads with one or more structures to reduce the likelihood of stimulation of unintended tissue.

### BACKGROUND

Malignant tachyarrhythmia, for example, ventricular fibrillation (VF), is an uncoordinated contraction of the cardiac muscle of the ventricles in the heart, and is the most commonly identified arrhythmia in cardiac arrest patients. If this arrhythmia continues for more than a few seconds, it may result in cardiogenic shock and cessation of effective blood circulation. As a consequence, sudden cardiac death (SCD) may result in a matter of minutes.

In patients with a high risk of VF, the use of implantable systems, such as an implantable cardioverter defibrillator (ICD) system has been shown to be beneficial at preventing SCD. Implantable systems, such as pacemakers with or without cardioversion or defibrillation capabilities, may also treat other cardiac dysfunction, such as bradycardia and heart failure. Such implantable systems may include electrical devices configured to deliver therapy via electrodes. Therapy may include shocks and/or anti-tachycardia pacing (ATP). The implantable systems may also be configured to deliver cardiac pacing to, for example, treat bradyarrhythmia or for cardiac resynchronization therapy (CRT).

The implantable system may include one or more implantable medical leads. A distal portion of an implantable medical lead may include one or more electrodes, and may be positioned at a target location within the patient for delivery of electrical therapy and/or electrical sensing via the electrodes. A proximal end of the lead may be coupled to the implantable system. The implantable system may also include one or more housing electrodes, which are sometimes referred to as can electrodes, for delivery of therapy and/or sensing.

Owing to the inherent surgical risks in attaching and replacing implantable medical leads directly within or on the heart, subcutaneous implantable systems have been devised, in which the implantable system and leads are located subcutaneously outside of the thorax. It has also been proposed that the distal portion of a lead of an implantable system may be implanted within the thorax, but not in contact with the heart, e.g., substernally. Additionally, it has been proposed to implant the distal portion of a lead of an implantable system within an extracardiac vessel that is within the thorax, such as the internal thoracic vein (ITV), the intercostal veins, the superior epigastric vein, or the azygos, hemiazygos, and accessory hemiazygos veins.

Implantable medical leads are also used to deliver therapies to tissues other than the heart. Implantable medical leads may be used to position one or more electrodes within or near target nerves, muscles, or organs to deliver electrical stimulation to such tissues. As examples, implantable medical leads may be positioned in the epidural space to deliver spinal cord stimulation, or proximate to other nerves, such as pelvic nerves or renal nerves, to deliver neurostimulation to the nerves. US 2021/268297 A1 relates to an implantable medical lead with a shield.

### SUMMARY

Relative to electrodes on or within the heart, delivery of pacing pulses using electrodes of extravascular leads may require higher energy levels to provide therapy (e.g., pacing pulses to the heart). Furthermore, some pacing electrodes placed extravascularly may direct a significant portion of the electrical field produced by a pacing pulse away from the heart. The electrical field directed away from the heart may stimulate extracardiac tissue, such as the phrenic nerve, nerve endings in the intercostal regions, or other sensory or motor nerves. These issues may similarly occur when electrodes are implanted within extracardiac vessels within the thorax, such as the internal thoracic vein (ITV), the intercostal veins, the superior epigastric vein, or the azygos, hemiazygos, and accessory hemiazygos veins, or when electrodes are implanted in other extracardiac locations.

This disclosure describes implantable medical leads and implantable systems, such as implantable cardioverter defibrillator (ICD) systems, utilizing the leads. For example, this disclosure describes implantable medical leads that include a shield configured to impede the electric field from a pacing pulse, e.g., block or reduce the electric field, in a direction from the pacing electrode, away from the heart, e.g., an anterior direction and a conductive surface disposed on the shield to reduce a resistance of the pacing electrode and expand an electric field generated by the pacing electrode. In this manner, the shield may reduce the likelihood that pacing pulses delivered via the pacing electrode stimulate extracardiac tissue, such as sensory or motor nerves, which may reduce pain or other sensations associated with capture of such tissue and the conductive surface disposed on the shield to reduce a resistance of the pacing electrode and expand an electric field generated by the pacing electrode. Furthermore, the shield may direct the electrical field toward the heart, allowing lower energy level pacing pulses to capture the heart than may be required without the shield. Lower energy pacing pulses may also reduce the likelihood that pacing pulses delivered via the pacing electrode stimulate extracardiac tissue, and may result is less consumption of the power source of the ICD and, consequently, longer service life for the ICD.

Although described herein primarily in the context of ICD systems, various aspects of the techniques of this disclosure may be applied to implantable systems other than ICD systems, including, but not limited to, bradycardia or cardiac resynchronization therapy (CRT) pacemaker systems. Accordingly, implantable medical leads having one or more shields may be used in contexts other than that of ICD systems, both cardiac and non-cardiac. For example, implantable medical leads that have a shield over a portion of a surface of an electrode and the conductive surface disposed on the shield may be used with an extracardiac pacemaker system without defibrillation capabilities. In some examples, implantable medical leads may include a shield over a portion of a surface of an electrode to impede an electrical field resulting from delivery of neurostimulation from the electrode in a direction away from a target nerve and a conductive surface disposed on the shield to expand an electric field generated by the pacing electrode. In this manner, the shield may direct the neurostimulation to intended tissue, and reduce the likelihood that the neurostimulation stimulates unintended tissues while reducing an energy consumption of the power source of the ICD.

In one example, an implantable medical lead includes a first defibrillation electrode and a second defibrillation electrode. The implantable medical lead further includes a pacing electrode configured to deliver a pacing pulse that generates an electric field proximate to the pacing electrode. The implantable medical lead further includes a shield disposed over a portion of an outer surface of the pacing electrode and extending laterally away from the pacing electrode. The shield is configured to impede the electric field in a direction from the pacing electrode away from a heart. The implantable medical lead further includes a conductive surface disposed on the shield and electrically coupled to the pacing electrode.

In another example, an implantable medical system includes an implantable medical device comprising, a housing, and therapy delivery circuitry within the housing. The implantable medical system further includes an implantable medical lead configured to be coupled to the medical device including a first defibrillation electrode and a second defibrillation electrode, a pacing electrode configured to deliver a pacing pulse that generates an electric field proximate to the pacing electrode, and a shield disposed over a portion of an outer surface of the pacing electrode and extending laterally away from the pacing electrode. The shield is configured to impede the electric field in a direction from the pacing electrode away from a heart. The implantable medical lead further includes a conductive surface disposed on the shield and electrically coupled to the pacing electrode.

This summary is intended to provide an overview of the subject matter described in this disclosure. It is not intended to provide an exclusive or exhaustive explanation of the systems, devices, and methods described in detail within the accompanying drawings and description below. Further details of one or more examples are set forth in the accompanying drawings and the description below. Other features, objects, and advantages will be apparent from the description and drawings, and from the statements provided below.

### BRIEF DESCRIPTION OF DRAWINGS

FIG. 1A is a front view of a patient implanted with the extracardiovascular ICD system implanted intra-thoracically.
FIG. 1B is a side view of the patient implanted with the extracardiovascular ICD system implanted intra-thoracically.
FIG. 1C is a transverse view of the patient implanted with the extracardiovascular ICD system implanted intra-thoracically.
FIG. 2A is a conceptual diagram of an example lead with a shield.
FIG. 2B is a conceptual diagram of an example simulated current density for pacing the electrode of FIG. 2A.
FIG. 3A is a conceptual diagram of an example lead with a disk-shaped shielded electrode, in accordance with techniques described herein.
FIG. 3B is a conceptual diagram of an example simulated current density for pacing the electrode of FIG. 3B.
FIG. 4 is a functional block diagram of an example configuration of electronic components of an example ICD.
FIGS. 5A, 5B are conceptual diagrams of a first example shielded electrode, in accordance with techniques described herein.
FIG. 6 is a conceptual diagram of second example shielded electrode, in accordance with techniques described herein.
FIG. 7 is a conceptual diagram of a third example shielded electrode, in accordance with techniques described herein.
FIG. 8 is a conceptual diagram of a kidney bean shaped shield, in accordance with techniques described herein.
FIG. 9 is a conceptual diagram of shielded electrode with a flexible wire in a spiral configuration, in accordance with techniques described herein.

### DETAILED DESCRIPTION

The pacing electrode in extravascular implantable cardioverter-defibrillator (EV ICD) may not be in direct contact with the heart tissue of a patient, which may result in a relatively high pacing voltage threshold as compared to the endo-or epicardial pacing electrodes. The high pacing threshold for EV ICD may be disadvantageous for EV ICD longevity.

In accordance with the techniques of the disclosure, the pacing electrode for an EV ICD may be configured to decrease the pacing voltage threshold. For example, a conductive surface may be disposed on the shield and electrically coupled to the pacing electrode, which may reduce a resistance of the pacing electrode and/or expand an electric field generated by the pacing electrode. Reducing the resistance of the pacing electrode and/or expanding the electric field generated by the pacing electrode may reduce an amount of current used to generate a pacing pulse, which may decrease an amount of power used by the EV ICD.

Techniques described herein may leverage the presence of non-conductive shield at the anterior side of the existing pacing electrode. This shield may be foldable so the EV ICD lead can be pushed through the introducer. This design may add a foldable conductive surface to the central area of this non-conductive shield (e.g., about 1/2 to 1/4 of a total shield area) on the posterior side of this non-conductive shield. This conductive surface may make an electrical contact with EV ICD pacing ring, which may effectively increase the pacing electrode surface. The current densities at the heart surface (e.g., to capture the heart) and at the edges of non-conductive shield (e.g., to prevent nerve sensation) remain the same for compared to systems that do not use the conductive surface. However, the pacing impedance when using the conductive surface may result in a lower pacing voltage and lower pacing energy thresholds compared to EV ICD systems that do not use a conductive surface disposed on a shield. In this way, techniques described herein may decrease a EV ICD pacing threshold by making electrically conductive the central portion of the shield (e.g., a foldable isolation shield), which may effectively increase the surface area of EV ICD pacing electrode and lower a pacing voltage and pacing energy thresholds while potentially preserving the current density at the heart and at the muscle nerves.

As used herein, relational terms, such as "first" and "second," "over" and "under," "front" and "rear," and the like, may be used solely to distinguish one entity or element from another entity or element without necessarily requiring or implying any physical or logical relationship or order between such entities or elements.

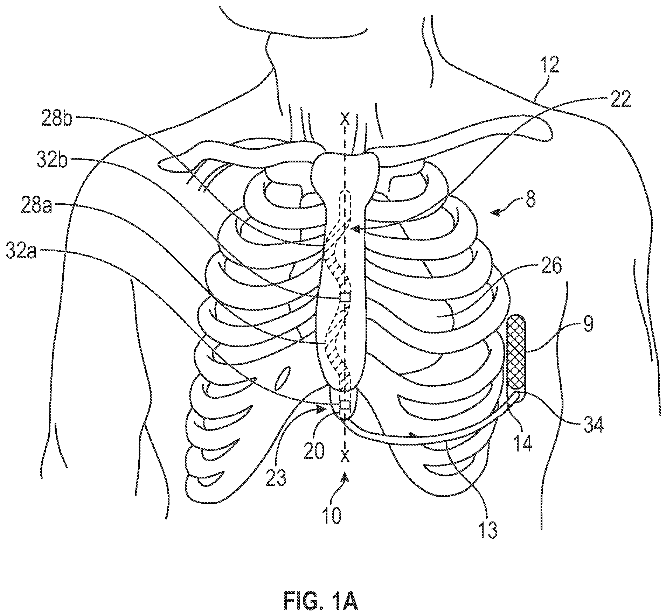

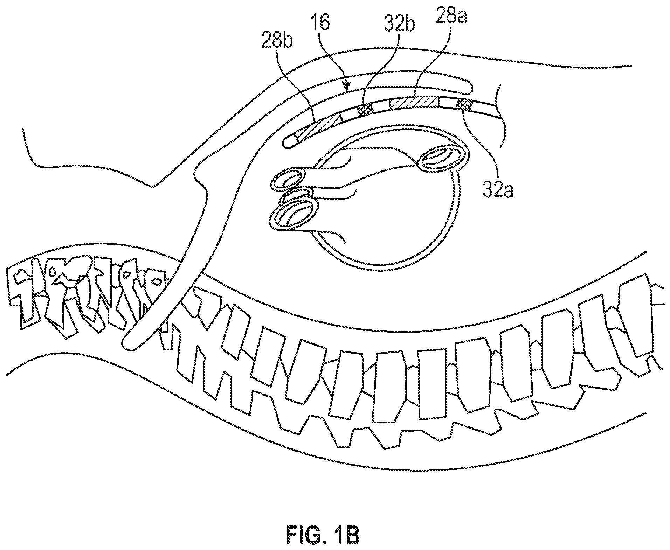

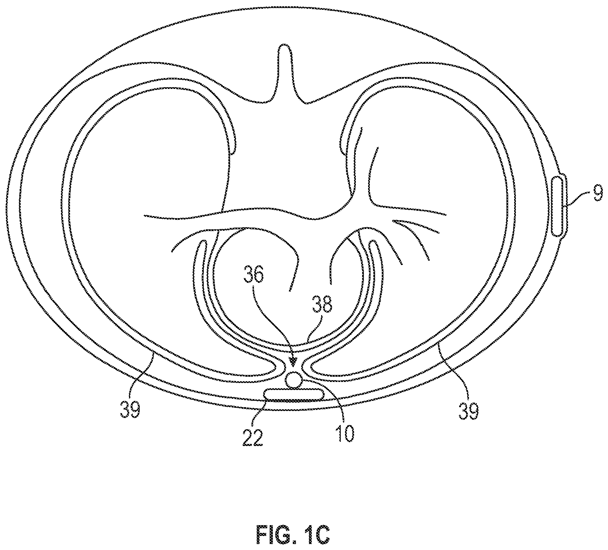

FIG. 1A is a front view of a patient 12 implanted with the extracardiovascular ICD system 8 implanted intra-thoracically. Referring now to the drawings in which like reference designators refer to like elements, there is shown in FIGS. 1A-1C conceptual diagrams illustrating various views of an example extracardiovascular ICD system 8. ICD system 8 includes an ICD 9 connected to an implantable medical lead 10. FIG. 1A is a front view of a patient implanted with extracardiovascular ICD system 8. FIG. 1B is a side view of the patient implanted with extracardiovascular ICD system 8. FIG. 1C is a transverse view of the patient implanted with extracardiovascular ICD system 8.

ICD 9 may include a housing that forms a hermetic seal that protects components of the ICD 9. The housing of ICD 9 may be formed of a conductive material, such as titanium or titanium alloy, which may function as a housing electrode (sometimes referred to as a can electrode). In some embodiments, ICD 9 may be formed to have or may include a plurality of electrodes on the housing. ICD 9 may also include a connector assembly (also referred to as a connector block or header) that includes electrical feedthroughs through which electrical connections are made between conductors of lead 10 and electronic components included within the housing of ICD 9. As will be described in further detail herein, the housing may house one or more processors, memories, transmitters, receivers, sensors, sensing circuitry, therapy circuitry, power sources and other appropriate components. The housing is configured to be implanted in a patient, such patient 12.

ICD 9 is implanted extra-thoracically on the left side of the patient, e.g., under the skin and outside the ribcage (subcutaneously or submuscularly). ICD 9 may, in some instances, be implanted between the left posterior axillary line and the left anterior axillary line of the patient. ICD 9 may, however, be implanted at other extra-thoracic locations on the patient as described later.

Lead 10 may include an elongated lead body 13 having a distal portion 16 sized to be implanted in an extracardiovascular location proximate the heart, e.g., intra-thoracically, as illustrated in FIGS. 1A-1C, or extra-thoracically. For example, lead 10 may extend extra-thoracically under the skin and outside the ribcage (e.g., subcutaneously or submuscularly) from ICD 9 toward the center of the torso of the patient, for example, toward the xiphoid process 23 of the patient. At a position proximate xiphoid process 23, the lead body 13 may bend or otherwise turn and extend superiorly. The bend may be pre-formed and/or lead body 13 may be flexible to facilitate bending. In the example illustrated in FIGS. 1A-1C, the lead body 13 extends superiorly intra-thoracically underneath the sternum, in a direction substantially parallel to the sternum.

Distal portion 16 of lead 10 may reside in a substernal location such that distal portion 16 of lead 10 extends superior along the posterior side of the sternum substantially within the anterior mediastinum 36. Anterior mediastinum 36 may be viewed as being bounded laterally by pleurae 39, posteriorly by pericardium 38, and anteriorly by the sternum 22. In some instances, the anterior wall of anterior mediastinum 36 may also be formed by the transversus thoracis and one or more costal cartilages. Anterior mediastinum 36 includes a quantity of loose connective tissue (such as areolar tissue), adipose tissue, some lymph vessels, lymph glands, substernal musculature (e.g., transverse thoracic muscle), the thymus gland, branches of the internal thoracic artery, and the ITV.

Lead body 13 may extend superiorly extra-thoracically (instead of intra-thoracically), e.g., either subcutaneously or submuscularly above the ribcage/sternum. Lead 10 may be implanted at other locations, such as over the sternum, offset to the right of the sternum, angled lateral from the proximal or distal end of the sternum, or the like. In some examples, lead 10 may be implanted within an extracardiac vessel within the thorax, such as the ITV, the intercostal veins, the superior epigastric vein, or the azygos, hemiazygos, and accessory hemiazygos veins. In some examples, distal portion 16 of lead 10 may be oriented differently than is illustrated in FIGS. 1A-1C, such as orthogonal or otherwise transverse to sternum 22 and/or inferior to heart 26. In such examples, distal portion 16 of lead 10 may be at least partially within anterior mediastinum 36. In some examples, distal portion 16 of lead 10 may be placed between the heart and lung as well as within the pleural cavity. In some examples, lead 10 may be implanted in the anterior mediastinum, intrapleurally, intrapericardially, epicardially, in the posterior mediastinum, and/or implanted through the intercostal space.

Lead body 13 may have a generally tubular or cylindrical shape and may define a diameter of approximately 3-9 French (Fr). However, lead bodies of less than 3 Fr and more than 9 Fr may also be utilized. In another configuration, lead body 13 may have a flat, ribbon, or paddle shape with solid, woven filament, or metal mesh structure, along at least a portion of the length of the lead body 13. In such an example, the width across lead body 13 may be between 1-3.5 mm. Other lead body designs may be used without departing from the scope of this application.

Lead body 13 may be formed from a non-conductive material, including silicone, polyurethane, fluoropolymers, mixtures thereof, and other appropriate materials, and shaped to form one or more lumens (not shown), however, the techniques are not limited to such constructions. Distal portion 16 may be fabricated to be biased in a desired configuration, or alternatively, may be manipulated by the user into the desired configuration. For example, the distal portion 16 may be composed of a malleable material such that the user can manipulate the distal portion into a desired configuration where it remains until manipulated to a different configuration.

Lead body 13 may include a proximal end 14 and a distal portion 16 which include electrodes configured to deliver electrical energy to the heart or sense electrical signals of the heart. Distal portion 16 may be anchored to a desired position within the patient, for example, substernally or subcutaneously by, for example, suturing distal portion 16 to the patient's musculature, tissue, or bone at the xiphoid process entry site. In some examples, distal portion 16 may be anchored to the patient or through the use of rigid tines, prongs, barbs, clips, screws, and/or other projecting elements or flanges, disks, pliant tines, flaps, porous structures such as a mesh-like elements and metallic or non-metallic scaffolds that facilitate tissue growth for engagement, bio-adhesive surfaces, and/or any other non-piercing elements.

Lead body 13 may define a substantially linear portion 20 (FIG. 1A) as it curves or bends near the xiphoid process 23 and extends superiorly. As shown in FIG. 1A, at least a part of distal portion 16 may define an undulating configuration distal to the substantially linear portion 20. In particular, distal portion 16 may define an undulating pattern, e.g., zig-zag, meandering, sinusoidal, serpentine, or other pattern, as it extends toward the distal end of lead 10. In other configurations, lead body 13 may not have a substantially linear portion 20 as it extends superiorly, but instead the undulating configuration may begin immediately after the bend.

Distal portion 16 includes one or more defibrillation electrodes configured to deliver an anti-tachyarrhythmia, e.g., cardioversion/defibrillation, shock to heart 26 of patient 12. In some examples, distal portion 16 includes a plurality of defibrillation electrodes spaced a distance apart from each other along the length of distal portion 16. In the example illustrated by FIGS. 1A-1C, distal portion 16 includes two defibrillation electrodes 28a and 28b (collectively, "defibrillation electrodes 28").

Defibrillation electrodes 28 may be disposed around or within the lead body 13 of the distal portion 16, or alternatively, may be embedded within the wall of the lead body 13. In one configuration, defibrillation electrodes 28 may be coil electrodes formed by a conductor. The conductor may be formed of one or more conductive polymers, ceramics, metal-polymer composites, semiconductors, metals or metal alloys, including but not limited to, one of a combination of the platinum, tantalum, titanium, niobium, zirconium, ruthenium, indium, gold, palladium, iron, zinc, silver, nickel, aluminum, molybdenum, stainless steel, MP35N, carbon, copper, polyaniline, polypyrrole, and other polymers. In another configuration, each of defibrillation electrodes 28 may be a flat ribbon electrode, a paddle electrode, a braided or woven electrode, a mesh electrode, a directional electrode, a patch electrode or another type of electrode configured to deliver a cardioversion/defibrillation shock to heart 26 of patient 12.

Defibrillation electrodes 28 may be electrically connected to one or more conductors, which may be disposed in the body wall of lead body 13 or in one or more insulated lumens (not shown) defined by lead body 13. In an example configuration, each of defibrillation electrodes 28 is connected to a common conductor such that a voltage may be applied simultaneously to all defibrillation electrodes 28 to deliver an anti-tachyarrhythmia shock to heart 26. In other configurations, defibrillation electrodes 28 may be attached to separate conductors such that each defibrillation electrode 28 may apply a voltage independent of the other defibrillation electrodes 28. In this case, ICD 9 or lead 10 may include one or more switches or other mechanisms to electrically connect the defibrillation electrodes together to function as a common polarity electrode such that a voltage may be applied simultaneously to all defibrillation electrodes 28 in addition to being able to independently apply a voltage.

Distal portion 16 may also include one or more pacing and/or sensing electrodes configured to deliver pacing pulses to heart 26 and/or sense electrical activity of heart 26. Such electrodes may be referred to as pacing electrodes, sensing electrodes, or pace/sense electrodes. In the example illustrated by FIGS. 1A-1C, distal portion 16 includes two pace/sense electrodes 32a and 32b (collectively, "pace/sense electrodes 32").

In the illustrated example of FIGS. 1A-1C, pace/sense electrode 32b is positioned between defibrillation electrodes 28, e.g., within a gap between the defibrillation electrodes, and pace/sense electrode 32a is positioned more proximal along distal portion 16 than proximal defibrillation electrode 28a. In some examples, more than one electrode 32 may exist within the gap between defibrillation electrodes 28. In some examples, an electrode 32 is additionally or alternatively located distal of the distalmost defibrillation electrode 28b.

Electrodes 32 may be configured to deliver low-voltage electrical pulses to the heart or may sense a cardiac electrical activity, e.g., depolarization and repolarization of the heart. As such, electrodes 32 may be referred to herein as pace/sense electrodes 32. In one configuration, electrodes 32 are ring electrodes. However, in other configurations electrodes 32 may be any of a number of different types of electrodes, including ring electrodes, short coil electrodes, paddle electrodes, hemispherical electrodes, or directional electrodes. Each of electrodes 32 may be the same or different types of electrodes as others of electrodes 32. Electrodes 32 may be electrically isolated from an adjacent defibrillation electrode 28 by including an electrically insulating layer of material between electrodes 32 and adjacent defibrillation electrodes 28. Each electrode 32 may have its own separate conductor such that a voltage may be applied to or sensed via each electrode independently from another electrode 32.

Electrodes 28 are referred to as defibrillation electrodes, and electrodes 32 are referred to as pace/sense electrodes, because they may have different physical structures enabling different functionality. Defibrillation electrodes 28 may be larger, e.g., have greater surface area, than pace/sense electrodes 32 and, consequently, may be configured to deliver anti-tachyarrhythmia shocks that have relatively higher voltages than pacing pulses. The relatively smaller size of pace/sense electrodes 32 may provide advantages over defibrillation electrodes for delivering pacing pulses and sensing intrinsic cardiac activity, e.g., lower pacing capture thresholds and/or better sensed signal quality. Nevertheless, a defibrillation electrode 28 may be used to deliver pacing pulses and/or sense electrical activity of the heart, such as in combination with a pace/sense electrode 32.

In the configuration shown in FIGS. 1A-1C, each electrode 32 is substantially aligned along a major longitudinal axis ("x"). In one example, the major longitudinal axis is defined by a portion of elongate body 12, e.g., substantially linear portion 20. In another example, the major longitudinal axis is defined relative to the body of the patient, e.g., along the anterior median line (or midsternal line), one of the sternal lines (or lateral sternal lines), left parasternal line, or other line.

In one configuration, the midpoint of each electrode 32a and 32b is along the major longitudinal axis "x," such that each electrode 32a and 32b is at least disposed at substantially the same horizontal position when the distal portion is implanted within the patient. In some examples, the longitudinal axis "x" may correspond to a caudal-cranial axis of the patient and a horizontal axis orthogonal to the longitudinal axis "x" may correspond to a medial-lateral axis of the patient. In other configurations, the electrodes 32 may be disposed at any longitudinal or horizontal position along the distal portion 16 disposed between, proximal to, or distal to the defibrillation electrodes 28. In the example illustrated in FIG. 1A, electrodes 32 are disposed along the undulating configuration of distal portion 16 at locations that will be closer to heart 26 of patient 12 than defibrillation electrodes 28 (e.g., at a peak of the undulating configuration that is toward the left side of the sternum). As illustrated in FIG. 1A, for example, electrodes 32 are substantially aligned with one another along the left sternal line. In the example illustrated in FIG. 1A, defibrillation electrodes 28 are disposed along peaks of the undulating configuration that extend toward a right side of the sternum away from the heart. This configuration places pace/sense electrodes 32 at locations closer to the heart than electrodes 28, to facilitate cardiac pacing and sensing at relatively lower amplitudes.

In some examples, pace/sense electrodes 32 and the defibrillation electrodes 28 may be disposed in a common plane when distal portion 16 is implanted extracardiovasculalry. In other configurations, the undulating configuration may not be substantially disposed in a common plane. For example, distal portion 16 may define a concavity or a curvature.

Proximal end 14 of lead body 13 may include one or more connectors 34 to electrically couple lead 10 to ICD 9. ICD 9 may also include a connector assembly that includes electrical feedthroughs through which electrical connections are made between the one or more connectors 34 of lead 10 and the electronic components included within the housing. The housing of ICD 9 may house one or more processors, memories, transmitters, receivers, sensors, sensing circuitry, therapy circuitry, power sources (e.g., capacitors and batteries), and/or other components. The components of ICD 9 may generate and deliver electrical therapy such as anti-tachycardia pacing, cardioversion or defibrillation shocks, post-shock pacing, and/or bradycardia pacing.

The undulating configuration of distal portion 16 and the inclusion of electrodes 32 between defibrillation electrodes 28 may provide a number of therapy vectors for the delivery of electrical therapy to the heart. For example, at least a portion of defibrillation electrodes 28 and one of electrodes 32 may be disposed over the right ventricle, or any chamber of the heart, such that pacing pulses and anti-tachyarrhythmia shocks may be delivered to the heart. The housing of ICD 9 may be charged with or function as a polarity different than the polarity of the one or more defibrillation electrodes 28 and/or electrodes 32 such that electrical energy may be delivered between the housing and the defibrillation electrode 28 and/or electrode 32 to the heart.

Each defibrillation electrode 28 may have the same polarity as every other defibrillation electrode 28 when a voltage is applied to it such that a shock may be delivered from all defibrillation electrodes together. In examples in which defibrillation electrodes 28 are electrically connected to a common conductor within lead body 13, this is the only configuration of defibrillation electrodes 28. However, in other examples, defibrillation electrodes 28 may be coupled to separate conductors within lead body 13 and may therefore each have different polarities such that electrical energy may flow between defibrillation electrodes 28, or between one of defibrillation electrodes 28 and one of pace/sense electrodes 32 or the housing electrode, to provide anti-tachyarrhythmia shock, pacing therapy, and/or to sense cardiac depolarizations. In this case, defibrillation electrodes 28 may still be electrically coupled together, e.g., via one or more switches within ICD 9, to have the same polarity.

In some examples, distal portion 16 of lead 10 may include one or more shields. The shield or shields may be configured to impede an electric field from delivery of an electrical therapy via an electrode, e.g., from a pacing pulse, in a direction from the electrode away from the heart, e.g., in an anterior direction. In this manner, the shield may reduce the likelihood that the electrical field will stimulate extracardiac tissue, such as sensory or motor nerves. Furthermore, the shield may direct the electrical field toward the heart, allowing lower energy level pacing pulses to capture the heart than may be required without the shield. Lower energy pacing pulses may also reduce the likelihood that pacing pulses delivered via the pacing electrode stimulate extracardiac tissue, and may result in less consumption of the power source of ICD 9 and, consequently, longer service life for the ICD. The techniques of this disclosure may be applied to implantable systems other than ICD 9, including, but not limited to, bradycardia pacemaker systems. For example, a lead that does not include defibrillation electrodes may include one or more shields and may be used with a pacemaker system without defibrillation capabilities.

In accordance with the techniques of the disclosure, the pacing electrode of pace/sense electrodes 32 may be configured to decrease the pacing voltage threshold. For example, a conductive surface may be disposed on a shield and electrically coupled to the pacing electrode, which may reduce a resistance of the pacing electrode and/or expand an electric field generated by the pacing electrode. Reducing the resistance of the pacing electrode and/or expanding the electric field generated by the pacing electrode may reduce an amount of current used to generate a pacing pulse, which may decrease an amount of power used by ICD 9.

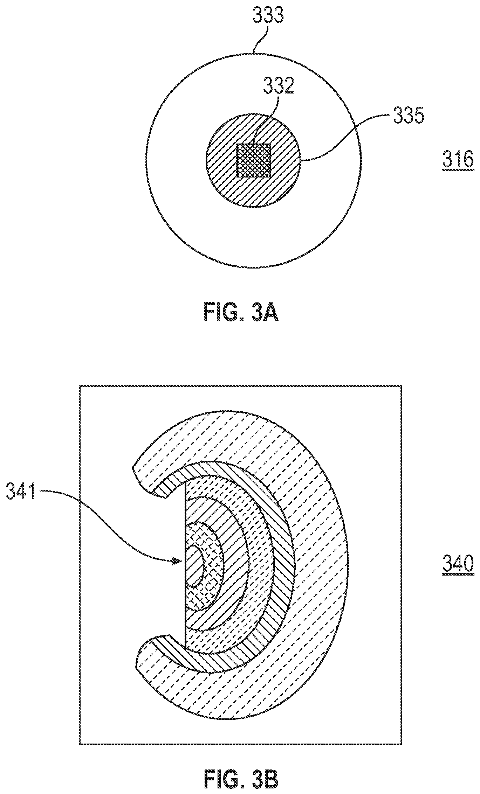

For example, lead 10 may include a first defibrillation electrode 28A and a second defibrillation electrode 28B that are configured to deliver anti tachyarrhythmia shocks. In this example, pacing electrode 32B may be configured to deliver a pacing pulse that generates an electric field proximate to the pacing electrode. A shield may be disposed over at least a portion of an outer surface of pacing electrode 32B, and extending laterally away from the pacing electrode 32B. The shield may be configured to impede the electric field in a direction from pacing electrode 32B away from the heart. In some examples, the shield may be disposed between first defibrillation electrode 28A and second defibrillation electrode 28B. A conductive surface may be disposed on the shield and electrically coupled to pacing electrode 32B. Further details of the shield and conductive surface are discussed with respect to FIGS. 3A, 3B.

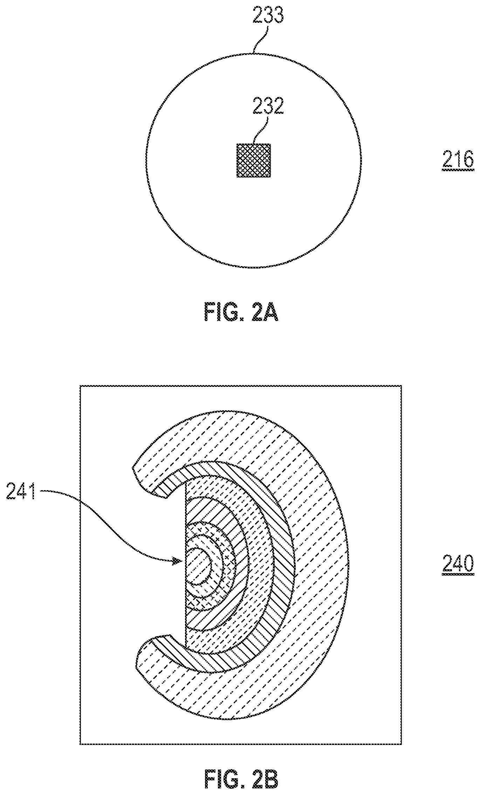

FIG. 2A is a conceptual diagram of an example shield 233 and pacing electrode 232 is arranged within shield 233, which may be included as part of a lead, like lead 10. Pacing electrode 232 in an extravascular implantable cardioverter-defibrillator (EV ICD) system may not be in direct contact with the heart tissue of a patient, which may result in a relatively high pacing voltage threshold (e.g, more than 8 V) and/or a relatively high resistance (e.g., more than 200 Ohms) as compared to the endo-or epicardial pacing electrodes. The relatively high pacing voltage threshold and/or a relatively high resistance for delivery of pacing by an EV ICD may be detrimental for EV ICD longevity.

FIG. 2B is a conceptual diagram of an example simulated current density 240 for pacing the electrode of FIG. 2A. In this example, a high current density 241 occurs at a position corresponding to the pacing electrode 232.

FIG. 3A is a conceptual diagram of an example disk-shaped shielded electrode, which may be included as part of a lead, like lead 10, in accordance with techniques described herein. In this example, a pacing electrode 332 is arranged within shield 333 and electrically coupled to a conductive surface 335. Shield 333 may be a non-conductive shield. For example, shield 333 may be formed of a polyurethane polymer and/or silicone. While shield 333 is circular in the example of FIG. 3A, shield 333 may be, for example, elliptical, kidney bean-shaped, umbrella-shaped, or another geometry.

Conductive surface 335 may include a foldable conductive surface. For example, conductive surface 335 may cover more than 25 % of a total surface area of a side of shield 333. In some examples, conductive surface 335 may cover more than 25 % of the total surface area of the side of shield 333 and cover less than 50 % of the total surface area of the side of shield 333. Conductive surface 335 may cover more than 10 % of the total surface area of the side of shield 333 and cover less than 75 % of the total surface area of the side of shield 333. In some examples, conductive surface 335 may cover 100 % of the total surface area of the side of shield 333. Conductive surface 335 may be formed of one or more of a foldable wire, a set of graphene tubes, or a conductive mesh. While conductive surface 335 is circular in the example of FIG. 3A, conductive surface 335 may be, for example, elliptical, kidney bean-shaped, umbrella-shaped, or another geometry, which may, but need not necessarily correspond to a geometry of shield 333.

In accordance with the techniques of the disclosure, conductive surface 335 may be configured to decrease the pacing voltage threshold (e.g., to 2 V or less). For example, conductive surface 335 may be disposed on shield 333 and electrically coupled to pacing electrode 332, which may reduce a resistance (e.g., 50 Ohms or less) of pacing electrode 332 and/or expand an electric field generated by pacing electrode 332. Reducing the resistance of pacing electrode 332 and/or expanding the electric field generated by pacing electrode 332 may reduce an amount of current used to generate a pacing pulse, which may decrease an amount of power used by ICD 9 (e.g., 4 times pacing energy savings from the example of FIG. 2A).

FIG. 3B is a conceptual diagram of an example simulated current density 340 for pacing the electrode of FIG. 3B. In the example of 3B, a relatively low current density 341 occurs at a position corresponding to the pacing electrode 332 and conductive surface 335. In this way, techniques described herein may decrease a pacing threshold by making electrically conductive the central portion of the shield (e.g., a foldable isolation shield), which may effectively increase the surface area of pacing electrode 332 and lower a pacing voltage and pacing energy thresholds while potentially preserving the current density at the heart and at the muscle nerves.

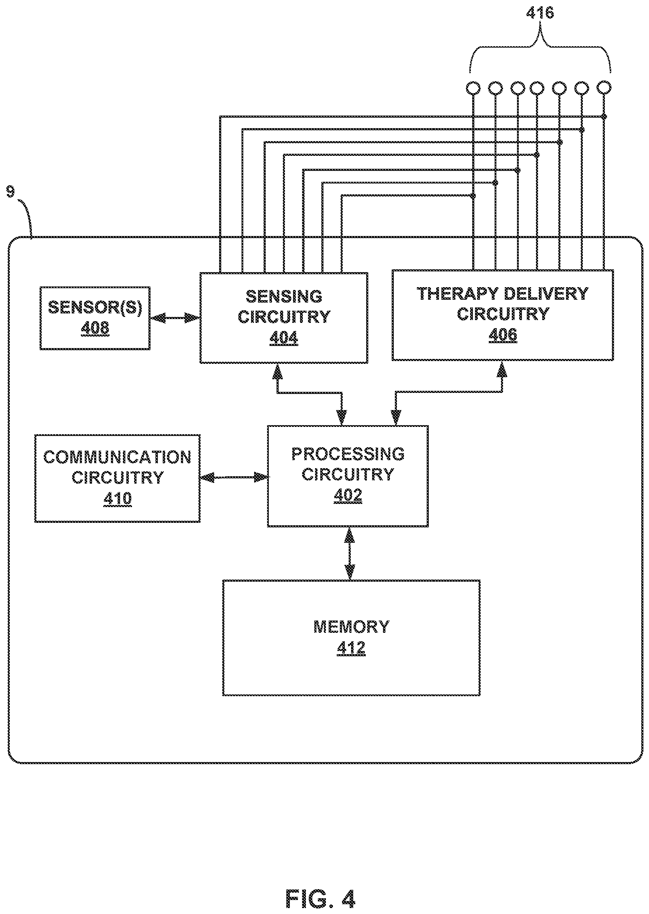

FIG. 4 is a functional block diagram of an example configuration of electronic components and other components of ICD 9. ICD 9 includes a processing circuitry 402, sensing circuitry 404, therapy delivery circuitry 406, sensors 408, communication circuitry 410, and memory 412. In some examples, ICD 9 may include more or fewer components. The described circuitry and other components may be implemented together on a common hardware component or separately as discrete but interoperable hardware or software components. Depiction of different features is intended to highlight different functional aspects and does not necessarily imply that such circuitry and other components must be realized by separate hardware or software components. Rather, functionality associated with one or more circuitries and components may be performed by separate hardware or software components, or integrated within common or separate hardware or software components.

Sensing circuitry 404 may be electrically coupled to some or all of electrodes 416, which may correspond to any of the defibrillation, pace/sense, and housing electrodes described herein. Sensing circuitry 404 is configured to obtain signals sensed via one or more combinations of electrodes 416 and process the obtained signals.

The components of sensing circuitry 404 may be analog components, digital components or a combination thereof. Sensing circuitry 404 may, for example, include one or more sense amplifiers, filters, rectifiers, threshold detectors, analog-to-digital converters (ADCs) or the like. Sensing circuitry 404 may convert the sensed signals to digital form and provide the digital signals to processing circuitry 402 for processing or analysis. For example, sensing circuitry 404 may amplify signals from the sensing electrodes and convert the amplified signals to multi-bit digital signals by an ADC. Sensing circuitry 404 may also compare processed signals to a threshold to detect the existence of atrial or ventricular depolarizations (e.g., P- or R waves) and indicate the existence of the atrial depolarization (e.g., P-waves) or ventricular depolarizations (e.g., R-waves) to processing circuitry 402. As shown in FIG. 4, ICD 9 may additionally include one or more sensors 408, such as one or more accelerometers, which may be configured to provide signals indicative of other parameters of a patient, such as activity or posture, to processing circuitry 402.

Processing circuitry 402 may process the signals from sensing circuitry 404 to monitor electrical activity of heart 26 of patient 12. Processing circuitry 402 may store signals obtained by sensing circuitry 404 as well as any generated EGM waveforms, marker channel data or other data derived based on the sensed signals in memory 412. Processing circuitry 402 may analyze the EGM waveforms and/or marker channel data to detect arrhythmias (e.g., bradycardia or tachycardia). In response to detecting the cardiac event, processing circuitry 402 may control therapy delivery circuitry 406 to deliver the desired therapy to treat the cardiac event, e.g., defibrillation shock, cardioversion shock, ATP, post shock pacing, or bradycardia pacing.

Therapy delivery circuitry 406 is configured to generate and deliver electrical therapy to heart 26. Therapy delivery circuitry 406 may include one or more pulse generators, capacitors, and/or other components capable of generating and/or storing energy to deliver as pacing therapy, defibrillation therapy, cardioversion therapy, cardiac resynchronization therapy, other therapy or a combination of therapies. In some instances, therapy delivery circuitry 406 may include a first set of components configured to provide pacing therapy and a second set of components configured to provide defibrillation therapy. In some instances, therapy delivery circuitry 406 may utilize the same set of components to provide both pacing and defibrillation therapy. In still other instances, therapy delivery circuitry 406 may share some of the defibrillation and pacing therapy components while using other components solely for defibrillation or pacing. Processing circuitry 402 may control therapy delivery circuitry 406 to deliver the generated therapy to heart 26 via one or more combinations of electrodes 416. Although not shown in FIG. 4, ICD 9 may include switching circuitry configurable by processing circuitry 402 to control which of electrodes 416 is connected to therapy delivery circuitry 406 and sensing circuitry 404.

Communication circuitry 410 may include any suitable hardware, firmware, software or any combination thereof for communicating with another device, such as a clinician programmer, a patient monitoring device, or the like. For example, communication circuitry 410 may include appropriate modulation, demodulation, frequency conversion, filtering, and amplifier components for transmission and reception of data with the aid of an antenna.

The various components of ICD 9 may include any one or more processors, controllers, digital signal processors (DSPs), application specific integrated circuits (ASICs), field-programmable gate arrays (FPGAs), or equivalent discrete or integrated circuitry, including analog circuitry, digital circuitry, or logic circuitry. Processing circuitry 402 may include fixed function circuitry and/or programmable processing circuitry. The functions attributed to processing circuitry 402 herein may be embodied as software, firmware, hardware or any combination thereof.

Memory 412 may include computer-readable instructions that, when executed by processing circuitry 402 or other components of ICD 9, cause one or more components of ICD 9 to perform various functions attributed to those components in this disclosure. Memory 412 may include any volatile, non-volatile, magnetic, optical, or electrical media, such as a random-access memory (RAM), read-only memory (ROM), non-volatile RAM (NVRAM), static non-volatile RAM (SRAM), electrically-erasable programmable ROM (EEPROM), flash memory, or any other non-transitory computer-readable storage media.

The leads and systems described herein may be used at least partially within the substernal space, e.g., within anterior mediastinum of patient, to provide an extravascular ICD system. An implanter (e.g., a physician) may implant the distal portion of the lead intra-thoracically using any of a number of implant tools, e.g., tunneling rod, sheath, or other tool that can traverse the diagrammatic attachments and form a tunnel in the substernal location. For example, the implanter may create an incision near the center of the torso of the patient, e.g., and introduce the implant tool into the substernal location via the incision. The implant tool is advanced from the incision superior along the posterior of the sternum in the substernal location. The distal portion of the lead is introduced into the tunnel via implant tool (e.g., via a sheath). As the distal portion is advanced through the substernal tunnel, the distal portion is relatively straight. The pre-formed or shaped undulating configuration is flexible enough to be straightened out while routing the lead through a sheath or other lumen or channel of the implant tool. Once the distal portion is in place, the implant tool is withdrawn toward the incision and removed from the body of the patient while leaving the lead in place along the substernal path. As the implant tool is withdrawn, the distal end of the lead takes on its pre-formed undulating configuration, and the shield transitions to its deployed configuration. In some examples, the shield is configured to be folded or wrapped around the pacing electrode for delivery via a lumen of an implant tool, and configured to be opened via air passage from a lumen going from the shield to the connector (e.g., a balloon deployed shield).

In some examples, rather than extending in a superior direction along the sternum, the distal portion of the lead may be oriented orthogonal or otherwise transverse to the sternum and/or inferior to the heart. In such examples, the lead may include one or more shields that cover a portion of an outer surface of one or more electrodes, e.g., an anterior and/or inferior portion, according to any of the examples described herein. Such shield(s) may impede an electrical field in a direction away from the heart, which may be an anterior and/or inferior direction. In some examples, the distal portion of the lead may be placed between the heart and lung as well as within the pleural cavity. In some examples, the lead may be implanted in the anterior mediastinum intrapleurally, intrapericardially, epicardially, in the posterior mediastinum, and/or implanted through the intercostal space.

In accordance with the techniques of the disclosure, the pacing electrode of electrodes 416 may be configured to decrease the pacing voltage threshold. For example, a conductive surface may be disposed on a shield and electrically coupled to the pacing electrode, which may reduce a resistance of the pacing electrode and/or expand an electric field generated by the pacing electrode. Reducing the resistance of the pacing electrode and/or expanding the electric field generated by the pacing electrode may reduce an amount of current used to generate a pacing pulse, which may decrease an amount of power used by ICD 9.

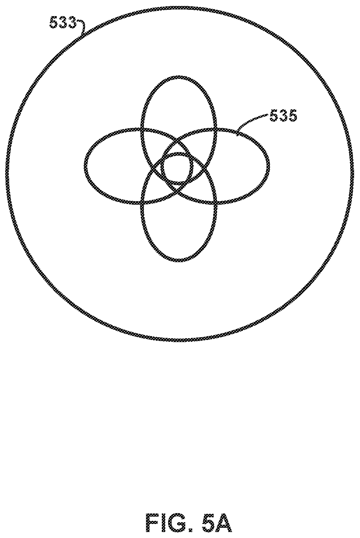

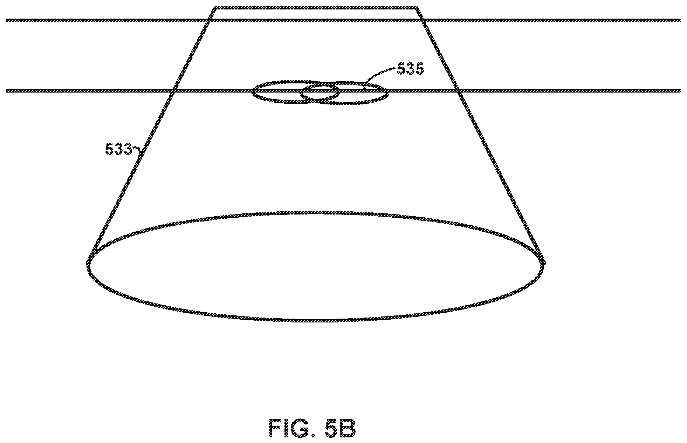

FIGS. 5A, 5B are conceptual diagrams of a first example shielded electrode, in accordance with techniques described herein. FIG. 5A is a front view of shield 533 and FIG. 5B is a side view of shield 533. In the example of FIGS. 5A, 5B, conductive surface 535 includes a foldable wire disposed on shield 533. The foldable wire of conductive surface 535 may be formed of, for example, platinum. As shown, the foldable wire of conductive surface 535 may be arranged to form petal shaped traces on shield 533.

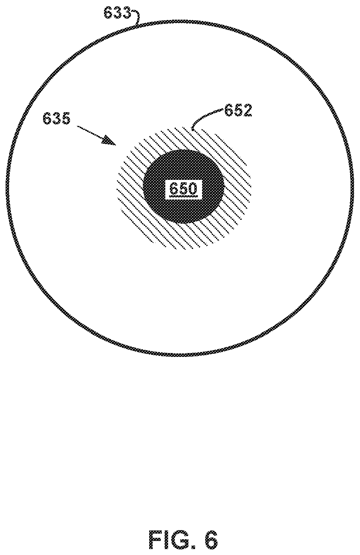

FIG. 6 is a conceptual diagram of second example shielded electrode, in accordance with techniques described herein. In the example of FIG. 6, conductive surface 635 is formed on shield 633 and includes a first portion 650 with a first conductivity and a second portion 652 with a second conductivity that is less than (e.g., or greater than) the first conductivity. As shown, second portion 652 forms a ring around first portion 635. Conductive surface 635 may be formed of, for example, a conductive polymer. Both first portion 650 and second portion 652 may be coupled to a same power source. For example, first portion 650 and second portion 652 may be both be electrically coupled to one electrode (e.g., one of electrodes 32 of FIGS. 1A-1C).

For example, first portion 650 may be formed at a first thickness (e.g., of a conductive polymer) and second portion 652 may be formed at a second thickness (e.g. of the conductive polymer), different than the first thickness. In some examples, first portion 650 may be formed of a first conductive material with a first conductivity and second portion 652 may be formed of a second conductive material with a second conductivity that is different from (e.g., greater than or less than) the first conductivity.

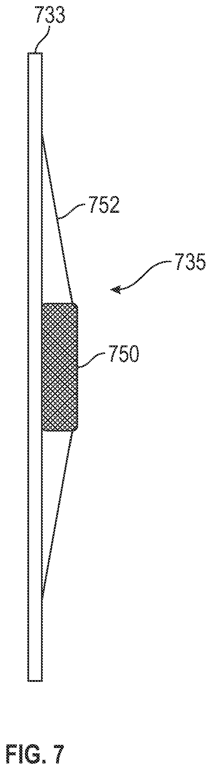

FIG. 7 is a conceptual diagram of a third example shielded electrode, in accordance with techniques described herein. In the example of FIG. 7, conductive surface 735 is formed on shield 733 and includes a first portion 750 with a first conductivity and a second portion 752 with a second conductivity that is less than the first conductivity. Conductive surface 735 may be formed of, for example, a conductive polymer. For example, first portion 750 may be formed at a first thickness (e.g., of a conductive polymer) and second portion 752 may be formed at a second thickness (e.g. of the conductive polymer).

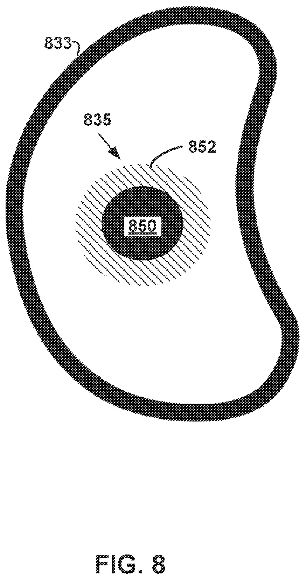

FIG. 8 is a conceptual diagram of a kidney bean shaped shield 833, in accordance with techniques described herein. In the example of FIG. 8, conductive surface 835 is formed on kidney bean shaped shield 833 and includes a first portion 850 with a first conductivity and a second portion 852 with a second conductivity that is less than the first conductivity.

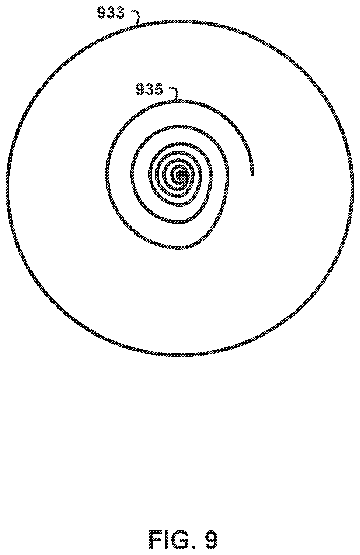

FIG. 9 is a conceptual diagram of shielded electrode with a flexible wire in a spiral configuration, in accordance with techniques described herein. In the example of FIG. 9, conductive surface 935 is formed on shield 833. As shown, the conductive surface comprises a foldable wire arranged in a spiral configuration. The foldable wire of conductive surface 935 may be formed of, for example, platinum.

It will be appreciated by persons skilled in the art that the present application is not limited to what has been particularly shown and described herein above. In addition, unless mention was made above to the contrary, it should be noted that all of the accompanying drawings are not to scale. A variety of modifications and variations are possible in light of the above teachings without departing from the scope of the invention, as defined by the following claims.

## Claims

1. An implantable medical lead comprising:
a first defibrillation electrode (28a) and a second defibrillation electrode (28b);
a pacing electrode (32) configured to deliver a pacing pulse that generates an electric field proximate to the pacing electrode;
a shield (333) disposed at least over a portion of an outer surface of the pacing electrode, and extending laterally away from the pacing electrode, wherein the shield is configured to impede the electric field in a direction from the pacing electrode away from a heart;
**characterized in that** it comprises
a conductive surface (335) disposed on the shield and electrically coupled to the pacing electrode.

2. The implantable medical lead of claim 1, wherein the shield is a non-conductive shield.

3. The implantable medical lead of any of claims 1-2, wherein the conductive surface is a foldable conductive surface.

4. The implantable medical lead of any of claims 1-3, wherein the conductive surface covers more than 25 % of a total surface area of a side of the shield, and more particularly the conductive surface covers more than 25 % of a total surface area of a side of the shield and less than 50 % of the total surface area of the side of the shield.

5. The implantable medical lead of any of claims 1-3, wherein the conductive surface covers more than 10 % of a total surface area of a side of the shield and less than 75 % of the total surface area of the side of the shield.

6. The implantable medical lead of any of claims 1-5, wherein the conductive surface comprises a foldable wire.

7. The implantable medical lead of claim 6, wherein the foldable wire is arranged in a spiral configuration or arranged to form petal shaped traces on the shield.

8. The implantable medical lead of any of claims 1-5, wherein the conductive surface comprises one or more of a conductive mesh and a set of graphene tubes.

9. The implantable medical lead of any of claims 1-8, wherein the conductive surface comprises a first portion with a first conductivity and a second portion with a second conductivity that is less than the first conductivity, wherein the second portion forms a ring around the first portion.

10. The implantable medical lead of any of claims 1-9, wherein the conductive surface comprises a conductive polymer, more particularly wherein the conductive surface comprises a first portion at a first thickness of the conductive polymer and a second portion at a second thickness of the conductive polymer.

11. The implantable medical lead of any of claims 1-10, wherein the conductive surface comprises a first portion formed of a first conductive material with a first conductivity and a second portion formed of a second conductive material with a second conductivity that is different from the first conductivity.

12. The implantable medical lead of any of claims 1-11, wherein the shield is configured to be folded or wrapped around the pacing electrode for delivery via a lumen of an implant tool, and configured to elastically unfold or unwrap to an open configuration when released from the lumen or opened via air passage from the lumen going from the shield.

13. The implantable medical lead of any of claims 1-12, wherein the portion of the surface of the pacing electrode is an anterior portion, and the shield is configured to impede the electric field in an anterior direction from the pacing electrode.

14. The implantable medical lead of any of claims 1-13, wherein the portion of the surface of the pacing electrode is an inferior portion, and the shield is configured to impede the electric field in an inferior direction from the pacing electrode.

15. An implantable medical system comprising:
an implantable medical device (9) comprising:
a housing; and
therapy delivery circuitry (406) within the housing; and
an implantable medical lead of any one of claims 1 - 14 and configured to be coupled to the implantable medical device.

## Patentansprüche

1. Implantierbare medizinische Leitung, umfassend:
eine erste Defibrillationselektrode (28a) und eine zweite Defibrillationselektrode (28b);
eine Schrittmacherelektrode (32), die dazu ausgestaltet ist, einen Schrittmacherimpuls zuzuführen, der ein elektrisches Feld nahe der Schrittmacherelektrode erzeugt;
eine Abschirmung (333), die zumindest über einem Abschnitt einer äußeren Oberfläche der Schrittmacherelektrode angeordnet ist und sich seitlich von der Schrittmacherelektrode weg erstreckt, wobei die Abschirmung dazu ausgestaltet ist, das elektrische Feld in einer von der Schrittmacherelektrode ausgehenden Richtung weg von dem Herzen zu behindern; **dadurch gekennzeichnet, dass** sie Folgendes umfasst
eine leitfähige Oberfläche (335), die auf der Abschirmung angeordnet und elektrisch mit der Schrittmacherelektrode gekoppelt ist.

2. Implantierbare medizinische Leitung nach Anspruch 1, wobei die Abschirmung eine nicht leitfähige Abschirmung ist.

3. Implantierbare medizinische Leitung nach einem der Ansprüche 1-2, wobei die leitfähige Oberfläche eine faltbare leitfähige Oberfläche ist.

4. Implantierbare medizinische Leitung nach einem der Ansprüche 1-3, wobei die leitfähige Oberfläche mehr als 25 % eines Gesamtoberflächenbereichs einer Seite der Abschirmung bedeckt und insbesondere die leitfähige Oberfläche mehr als 25 % eines Gesamtoberflächenbereichs einer Seite der Abschirmung und weniger als 50 % des Gesamtoberflächenbereichs der Seite der Abschirmung bedeckt.

5. Implantierbare medizinische Leitung nach einem der Ansprüche 1-3, wobei die leitfähige Oberfläche mehr als 10 % eines Gesamtoberflächenbereichs einer Seite der Abschirmung und weniger als 75 % des Gesamtoberflächenbereichs der Seite der Abschirmung bedeckt.

6. Implantierbare medizinische Leitung nach einem der Ansprüche 1-5, wobei die leitfähige Oberfläche einen faltbaren Draht umfasst.

7. Implantierbare medizinische Leitung nach Anspruch 6, wobei der faltbare Draht in einer spiralförmigen Ausgestaltung angeordnet oder so angeordnet ist, dass er blütenblattförmige Bahnen auf der Abschirmung bildet.

8. Implantierbare medizinische Leitung nach einem der Ansprüche 1-5, wobei die leitfähige Oberfläche eines oder mehrere eines leitfähigen Netzes und eines Satzes von Graphenröhrchen umfasst.

9. Implantierbare medizinische Leitung nach einem der Ansprüche 1-8, wobei die leitfähige Oberfläche einen ersten Abschnitt mit einer ersten Leitfähigkeit und einen zweiten Abschnitt mit einer zweiten Leitfähigkeit, die kleiner als die erste Leitfähigkeit ist, umfasst, wobei der zweite Abschnitt einen Ring um den ersten Abschnitt bildet.

10. Implantierbare medizinische Leitung nach einem der Ansprüche 1-9, wobei die leitfähige Oberfläche ein leitfähiges Polymer umfasst, insbesondere wobei die leitfähige Oberfläche einen ersten Abschnitt mit einer ersten Dicke des leitfähigen Polymers und einen zweiten Abschnitt mit einer zweiten Dicke des leitfähigen Polymers umfasst.

11. Implantierbare medizinische Leitung nach einem der Ansprüche 1-10, wobei die leitfähige Oberfläche einen ersten Abschnitt, der aus einem ersten leitfähigen Material mit einer ersten Leitfähigkeit gebildet ist, und einen zweiten Abschnitt, der aus einem zweiten leitfähigen Material mit einer zweiten Leitfähigkeit gebildet ist, die sich von der ersten Leitfähigkeit unterscheidet, umfasst.

12. Implantierbare medizinische Leitung nach einem der Ansprüche 1-11, wobei die Abschirmung dazu ausgestaltet ist, zur Zuführung über ein Lumen eines Implantationswerkzeugs um die Schrittmacherelektrode herum gefaltet oder gewickelt zu werden, und dazu ausgestaltet ist, sich elastisch in eine offene Ausgestaltung zu entfalten oder abzuwickeln, wenn sie aus dem Lumen freigegeben oder über einen Luftdurchlass aus dem Lumen, der von der Abschirmung ausgeht, geöffnet wird.

13. Implantierbare medizinische Leitung nach einem der Ansprüche 1-12, wobei der Abschnitt der Oberfläche der Schrittmacherelektrode ein vorderer Abschnitt ist und die Abschirmung dazu ausgestaltet ist, das elektrische Feld in einer von der Schrittmacherelektrode ausgehenden Richtung nach vorn zu behindern.

14. Implantierbare medizinische Leitung nach einem der Ansprüche 1-13, wobei der Abschnitt der Oberfläche der Schrittmacherelektrode ein unterer Abschnitt ist und die Abschirmung dazu ausgestaltet ist, das elektrische Feld in einer von der Schrittmacherelektrode ausgehenden Richtung nach unten zu behindern.

15. Implantierbares medizinisches System, umfassend:
eine implantierbare medizinische Vorrichtung (9), die Folgendes umfasst:
ein Gehäuse; und
eine Therapiezuführungsschaltung (406) innerhalb des Gehäuses; und
eine implantierbare medizinische Leitung nach einem der Ansprüche 1-14 und die dazu ausgestaltet ist, mit der implantierbaren medizinischen Vorrichtung gekoppelt zu sein.

## Revendications

1. Sonde médicale implantable comprenant :
une première électrode de défibrillation (28a) et une seconde électrode de défibrillation (28b) ;
une électrode de stimulation (32) configurée pour délivrer une impulsion de stimulation qui génère un champ électrique à proximité de l'électrode de stimulation ;
un blindage (333) disposé sur au moins une partie d'une surface extérieure de l'électrode de stimulation et s'étendant latéralement à partir de l'électrode de stimulation, le blindage étant configuré pour empêcher le champ électrique dans une direction s'éloignant de l'électrode de stimulation et d'un cœur, **caractérisé en ce qu'**il comprend
une surface conductrice (335) disposée sur le blindage et couplée électriquement à l'électrode de stimulation.

2. Conducteur médical implantable selon la revendication 1, le blindage étant un blindage non conducteur.

3. Conducteur médical implantable selon l'une quelconque des revendications 1 à 2, la surface conductrice étant une surface conductrice pliable.

4. Conducteur médical implantable selon l'une quelconque des revendications 1 à 3, la surface conductrice couvrant plus de 25 % d'une surface totale d'un côté du blindage, et plus particulièrement la surface conductrice couvrant plus de 25 % d'une surface totale d'un côté du blindage et moins de 50 % de la surface totale du côté du blindage.

5. Conducteur médical implantable selon l'une quelconque des revendications 1 à 3, la surface conductrice couvrant plus de 10 % d'une surface totale d'un côté du blindage et moins de 75 % de la surface totale du côté du blindage.

6. Conducteur médical implantable selon l'une quelconque des revendications 1 à 5, la surface conductrice comprenant un fil pliable.

7. Conducteur médical implantable selon la revendication 6, le fil pliable étant agencé dans une configuration en spirale ou agencé pour former des traces en forme de pétale sur le blindage.

8. Conducteur médical implantable selon l'une quelconque des revendications 1 à 5, la surface conductrice comprenant un ou plusieurs d'un maillage conducteur et d'un ensemble de tubes de graphène.

9. Conducteur médical implantable selon l'une quelconque des revendications 1 à 8, la surface conductrice comprenant une première partie avec une première conductivité et une seconde partie avec une seconde conductivité qui est inférieure à la première conductivité, la seconde partie formant un anneau autour de la première partie.

10. Conducteur médical implantable selon l'une quelconque des revendications 1 à 9, la surface conductrice comprenant un polymère conducteur, plus particulièrement la surface conductrice comprenant une première partie à une première épaisseur du polymère conducteur et une seconde partie à une seconde épaisseur du polymère conducteur.

11. Conducteur médical implantable selon l'une quelconque des revendications 1 à 10, la surface conductrice comprenant une première partie formée d'un premier matériau conducteur avec une première conductivité et une seconde partie formée d'un second matériau conducteur avec une seconde conductivité qui est différente de la première conductivité.

12. Sonde médicale implantable selon l'une quelconque des revendications 1 à 11, le blindage étant configuré pour être plié ou enroulé autour de l'électrode de stimulation afin d'être délivré par la lumière d'un outil d'implantation, et configuré pour se déplier ou se dérouler de manière élastique afin d'adopter une configuration ouverte lorsqu'il est libéré de la lumière ou ouvert passage d'air provenant de la lumière et s'étendant à partir du blindage.

13. Sonde médicale implantable selon l'une quelconque des revendications 1 à 12, la partie de la surface de l'électrode de stimulation étant une partie antérieure, et le blindage étant configuré pour empêcher le champ électrique dans une direction antérieure à partir de l'électrode de stimulation.

14. Sonde médicale implantable selon l'une quelconque des revendications 1 à 13, la partie de la surface de l'électrode de stimulation étant une partie inférieure, et le blindage étant configuré pour empêcher le champ électrique dans une direction inférieure à partir de l'électrode de stimulation.

15. Système médical implantable comprenant :
un dispositif médical implantable (9) comprenant :
un boîtier ; et
des circuits d'administration de thérapie (406) à l'intérieur du boîtier ; et
un conducteur médical implantable selon l'une quelconque des revendications 1 à 14 et configuré pour être couplé au dispositif médical implantable.
